**Europäisches Patentamt**

**European Patent Office**

**Office européen des brevets**

(19)

(11) Numéro de publication : **0 389 334 B1**

## FASCICULE DE BREVET EUROPEEN

(12)

(45) Date de publication du fascicule du brevet :
**22.09.93 Bulletin 93/38**

(51) Int. Cl.⁵ : **C07C 19/08, C07C 17/38**

(21) Numéro de dépôt : **90400664.0**

(22) Date de dépôt : **13.03.90**

(54) **Procédé de purification du 1,1,1,2-tétrafluoroéthane.**

(30) Priorité : **23.03.89 US 327640**

(43) Date de publication de la demande :
**26.09.90 Bulletin 90/39**

(45) Mention de la délivrance du brevet :
**22.09.93 Bulletin 93/38**

(84) Etats contractants désignés :
**DE ES FR GB GR IT NL**

(56) Documents cités :
**WO-A-90/10612**
**US-A- 2 863 830**
**US-A- 3 026 359**
**US-A- 4 129 603**

(73) Titulaire : **ELF ATOCHEM S.A.**
**4 & 8, Cours Michelet La Défense 10**
**F-92800 Puteaux (FR)**

(72) Inventeur : **Yates, Stephen Frederic**
**2140 Goebbert Road 4-210**
**Arlington Heights, Illinois 60005 (US)**

(74) Mandataire : **Leboulenger, Jean et al**
**ATOCHEM Département Propriété Industrielle**
**F-92091 Paris la Défense 10 Cédex 42 (FR)**

EP 0 389 334 B1

Jouve, 18, rue Saint-Denis, 75001 PARIS

## Description

La présente invention concerne la purification du 1,1,1,2-tétrafluoroéthane (connu aussi dans le métier sous la désignation R-134a) qui présente un intérêt particulier comme substitut aux chlorofluorocarbures à propriétés physiques similaires, en particulier le dichlorodifluorométhane (R-12).

Pour remplacer le composé R-12, suspecté de contribuer à l'affaiblissement de la couche d'ozone, on développe actuellement des procédés de production du R-134a. Ce composé peut être préparé par réaction d'autres chlorofluorocarbures avec HF ou des fluorures de métaux alcalins, en utilisant éventuellement des catalyseurs ou des moyens électrochimiques pour faciliter les réactions.

La fluoration d'un composé en $C_2$ pour produire du R-134a ne requiert pas seulement de nouveaux catalyseurs et des conditions opératoires appropriées. Il faut aussi considérer la récupération et la purification du R-134a, ces étapes dépendant des sous-produits formés par les réactions chimiques mises en oeuvre. Lorsqu'il quitte le réacteur où il est produit, le R-134a peut contenir de l'acide fluorhydrique non transformé, de l'acide chlorhydrique provenant de l'élimination des atomes de chlore du composé de départ et divers sous-produits contenant des nombres variables d'atomes d'hydrogène, de chlore et de fluor. Certains de ces sous-produits sont faciles à séparer par distillation ; d'autres sont relativement inoffensifs car leur présence n'altère pas sensiblement les propiétés physiques faisant l'utilité du R-134a. Bien qu'il ne soit présent qu'en quantités relativement petites dans le R-134a produit, le 2-chloro-1,1-difluoroéthylène (R-1122) est un sous-produit toxique qui doit être éliminé du R-134a avant son utilisation. Malheureusement, les points d'ébullition du R-1122 et du R-134a sont très proches (-17,1°C et -26,5°C respectivement) ; les deux composés ne sont donc pas efficacement séparables par distillation.

Dans le brevet US 3819493, Fozzard décrit un procédé de distillation extractive pour séparer le 1,1-difluoroéthane (R-152a) du R-134a produit par fluoration électrochimique du R-152a. Les deux composés ont une faible volatilité relative ; des hydrocarbures saturés en $C_4$-$C_{10}$ sont ajoutés pour accroître la volatilité et faciliter leur séparation.

Dans le brevet US 4129603, Bell élimine le R-1122 par mise en contact du R-134a impur avec une solution aqueuse d'un permanganate métallique. Le R-134a provient de la réaction de HF avec un haloéthane tel que le 2-chloro-1,1,1-trifluoroéthane, sur un catalyseur d'oxyde ou fluorure de chrome.

Une autre approche pour enlever le R-1122 du R-134a est décrite par Potter dans le brevet US 4158675. La réaction de production du R-134a est réalisée à des températures allant de 325 à 375°C en présence d'un catalyseur d'oxyde ou de fluorure de chrome. Potter fait passer l'effluent de la réaction dans un second réacteur contenant un catalyseur au chrome et travaillant à 100-275°C, et montre qu'on obtient ainsi une réduction substantielle du R-1122.

Les méthodes de l'art antérieur précité présentent cependant des aspects défavorables et il restait encore à améliorer les méthodes de purification du R-134a, en particulier pour éliminer le R-1122.

Il est bien sûr préférable qu'une impureté comme le R-1122 soit entièrement et sélectivement éliminée, non seulement du principal composé dans lequel il se trouve, c'est-à-dire le R-134a, mais aussi des autres sous-produits éventuellement présents. L'homme du métier comprendra qu'une telle élimination sélective est exceptionnelle. Dans la plupart des cas, l'élimination d'un composé indésirable spécifique requiert d'accepter aussi l'élimination d'autres composés. Cependant, si les composés éliminés sont indésirables dans le produit final, une adsorption dont la sélectivité n'est pas parfaite peut s'avérer utile. Par exemple, quand la méthode classique laisse de petites quantités de sous-produits, ceux-ci peuvent être éliminés dans une étape finale de purification.

Il a maintenant été trouvé que les charbons actifs sont capables d'éliminer presque complètement le R-1122 du R-134a et qu'en même temps ils éliminent aussi certaines des autres impuretés qu'on peut trouver dans un R-134a brut.

La présente invention a donc pour objet un procédé de purification d'un R-134a contenant environ 500 à 10000 ppm en poids de R-1122, consistant à mettre en contact ledit R-134a en phase liquide avec un charbon actif.

L'opération est de préférence réalisée à une température allant d'environ -10 à 100°C et une pression allant d'environ 100 à 860 kPa. Le R-134a récupéré peut contenir moins de 10 ppm en poids de R-1122.

Si on utilise un lit fixe de particules de charbon actif, le R-134a liquide peut être passé sur les particules à une vitesse spatiale horaire d'environ 1 à 30 hr$^{-1}$.

Les charbons actifs sont largement utilisés pour l'adsorption de matériaux indésirables à partir de vapeurs ou de liquides. Ils présentent une surface spécifique élevée et des pores dont les diamètres peuvent aller jusqu'à 30 angstroms et plus. Dérivant généralement de produits naturels, les charbons actifs ont une composition variable, même lorsqu'ils ont été carbonisés par chauffage pour éliminer les substances volatiles et ensuit oxydés dans la vapeur d'eau ou autres gaz à température élevée pour développer leurs porosité et surface spécifique. Certains charbons sont chimiquement activés durant l'opération de carbonisation. Les charbons actifs peuvent donc présenter des propriétés variables selon le matériau de départ et la méthode de fabrication.

Le R-134a peut provenir de l'hydrofluoration catalytique d'un composé en $C_2$ contenant des atomes de chlore qui sont remplacés par des atomes de fluor durant la réaction. La conversion en R-134a n'est pas totale et il se forme beaucoup de sous-produits, notamment HCl qui constitue un produit important de la réaction. Par conséquent, l'effluent du réacteur sera avantageusement distillé pour concentrer le R-134a formé et obtenir un recyclat de produit initial non converti. Le courant de R-134a brut ainsi obtenu contient généralement de l'acide fluorhydrique non transformé, de l'acide chlorhydrique, et des quantités mineures de différentes impuretés, notamment du R-1122. Les acides HF et HCl peuvent être séparés sélectivement de façon connue. Le R-134a ainsi obtenu contient encore des impuretés à éliminer, en particulier du R-1122 toxique. Le présent procédé est destiné à éliminer le R-1122 jusqu'à 10 ppm ou moins à partir d'un courant de R-134a concentré dèjà débarassé des acides HF et HCl, mais cela n'exclut pas nécessairement la présence d'un peu de HF et HCl.

Le courant de R-134a est traité en phase liquide pour éviter les coûts de vaporisation et de recondensation du courant. La mise en contact du courant de R-134a avec le charbon actif adsorbant peut être effectuée suivant les différentes techniques connues de l'homme du métier ; on peut opérer en lit fluide, mais on utilisera avantageusement un lit fixe de particules adsorbantes. Le choix de la dimension des particules, de la forme du lit et de la vitesse spatiale du courant de R-134a peut être déterminé suivant les principes connus de façon à obtenir l'élimination pratiquement totale du R-1122. D'une façon générale, pour un traitement en phase liquide, la vitesse spatiale horaire du courant R-134a devrait être d'environ 1 à 30 hr$^{-1}$.

L'adsorption doit être effectuée à une température convenable, généralement entre environ -10 et 100°C, et à une pression d'environ 100 à 860 kPa.

Le charbon actif adsorbant peut être régénéré par chauffage avec un courant gazeux pour désorber le R-1122 ; ce dernier peut éventuellement être recyclé au réacteur de fabrication du R-134a pour y être fluoré par HF. Une régénération satisfaisante du lit de charbon actif peut notamment être obtenue par chauffage à une température d'environ 100 à 200°C sous un courant d'azote. Après élimination du R-1122, le charbon actif est refroidi et remis en service.

Les exemples suivants illustrent l'invention sans la limiter.

## EXEMPLE 1 (comparatif)

Dans un tube d'acier inox de 9,5 mm de diamètre, maintenu à 25°C, on a placé 10 g d'un charbon actif du commerce. Sur cet adsorbant, on a fait passer à raison de 60 ml/min un courant type de R-134a partiellement purifié et on a déterminé la composition des gaz sortants par chromatographie gazeuse au moyen d'une colonne de 3048 mm de longueur et de 3,175 mm de diamètre contenant 1% de SP1000 sur un garnissage Carbopack B de 60-80 mesh (Supelco Inc.) fonctionnant à 45°C pendant 3 minutes et programmée ensuite pour atteindre 200°C à raison de 8°C/minute.

Le tableau A suivant donne les résultats d'une série d'essais réalisés avec six charbons actifs (PCB, BPL, CPG, OL, FCA et SGL) fournis par la Société CALGON.

## Tableau A

| Constituant (*) | Alimentation (ppm en poids) | PCB | BPL | CPG | OL | FCA | SGL |
|---|---|---|---|---|---|---|---|
| $CO_2$ | 17 | 18 | 16 | 13 | 9 | 12 | 11 |
| R-143a | 662 | 685 | 613 | 543 | 597 | 538 | 532 |
| R-12 | 22 | 9 | 15 | 19 | 0 | 11 | 12 |
| R-1122 | 1034 | 0 | 0 | 0 | 3 | 7 | 8 |
| R-124 | 166 | 0 | 52 | 43 | 0 | 120 | 30 |
| R-133a | 4166 | 0 | 6 | 13 | 9 | 309 | 62 |
| R-114a | 88 | 0 | 0 | 0 | 0 | 15 | 6 |
| R-134a | restant | - | - | - | - | - | - |

(*) R-143a : $CH_3CF_3$    R-124 : $CF_3CHClF$    R-134a : $CF_3CH_2F$
R-12 : $CCl_2F_2$    R-133a : $CF_3CH_2Cl$
R-1122 : $CF_2=CHCl$    R-114a : $CF_3CCl_2F$

On peut constater que l'objectif principal, à savoir l'élimination du R-1122 présent dans le R-134a, peut être atteint avec tous les charbons actifs testés, même si certains apparaissent supérieurs.

Aucun des charbons n'apparaît éliminer le $CO_2$ ou le R-134a. Le R-12 n'est éliminé que par le charbon OL. On notera que, puisque les quantités de certaines impuretés testées sont très faibles, on ne peut pas être sûr, sans une étude approfondie, que l'adsorption partielle apparente de ces constituants est significative. Cependant, lorsque comme dans le cas du R-1122 une quantité relativement importante des impuretés est complètement éliminée, on peut considérer que le résultat est significatif.

Les résultats obtenus en ce qui concerne les R-124 et R-133a sont variables. Les deux composés apparaissent être éliminés, mais l'origine du charbon actif semble influer grandement sur la quantité éliminée.

Tous les charbons apparaissent adsorber significativement le R-114a.

Parmi tous les charbons actifs testés, il semble que les charbons PCB et OL sont dans l'ensemble les plus efficaces.

Les charbons actifs testés présentent tous une surface spécifique relativement élevée (environ 1000-1200 m²/g) et la majeur partie de leur volume poreux est inférieure à environ 30 angstroms. Ils diffèrent par la quantité de surface spécifique associée à des pores supérieurs à 30 angstroms. Certains (PCB et BPL) sont destinés à une utilisation en phase vapeur, alors que d'autres (CPG, OL et SGL) sont destinés aux applications en phase liquide. On suppose que les charbons adsorbent les constituants testés en fonction de leurs points d'ébullition, les composés moins volatiles étant préférentiellement adsorbés. Cependant, les différences observées avec les différents charbons restent encore inexpliquées.

## EXEMPLE 2

Comme dans l'exemple 1, on a placé 10g de charbon actif CPG dans un tube d'acier inox de 9,5mm de diamètre. Pour cet essai, la température était d'environ 23°C et la pression relative était maintenue à 862 kPa de façon à ce que le R-134a soit en phase liquide au lieu d'être en phase vapeur comme à l'exemple 1. Un courant de R-134a partiellement purifié a été passé sur le charbon à raison de 0,5g/min et la composition des gaz sortants a été déterminée par chromatographie gazeuse comme à l'exemple 1. Les résultats figurent dans le tableau B suivant.

Tableau B

| Constituant | Alimentation (ppm en poids) | Effluent (ppm en poids) |
|---|---|---|
| $CO_2$ | 52 | 27 |
| R-143a | 5368 | 6341 |
| R-12 | 24 | 20 |
| R-1122 | 6082 | 99 |
| R-124 | 196 | 131 |
| R-133a | 413 | 266 |
| R-114a | 48 | 33 |

La composition d'alimentation comprenait nettement plus de R-143a et de R-1122, mais moins de R-133a. Les résultats sont nettement différents de ceux de l'exemple 1. Tandis que la plupart du R-1122 est éliminé conformément à l'objectif visé, l'adsorption des R-124, R-133a et R-114a est moindre qu'auparavant. Bien que dans cet essai l'effluent contienne plus que 10 ppm en poids de R-1122, il apparaît qu'un choix judicieux de la vitesse spatiale et du type de charbon actif peut permettre d'atteindre la concentration visée.

**Revendications**

1.  Procédé du purification du 1,1,1,2-tétrafluoroéthane (R-134a) contenant d'environ 500 à 10000 ppm en poids de 2-chloro-1,1-difluoroéthylène (R-1122), caractérisé par la mise en contact dudit 1,1,1,2-tétrafluoroéthane en phase liquide avec un charbon actif.

2.  Procédé selon la revendication 1, dans lequel on opère à une température allant d'environ -10 à 100°C et une pression allant d'environ 100 à 860 kPa.

3.  Procédé selon la revendication 1 ou 2, dans lequel, après ladite mise en contact, on récupère un R-134a contenant moins de 10 ppm en poids de 2-chloro-1,1-difluoroéthylène.

4.  Procédé selon l'une des revendication 1 à 3, dans lequel le charbon actif est utilisé sous forme d'un lit fixe de particules et la vitesse spatiale horaire dudit 1,1,1,2-tétrafluoroéthane est d'environ 1 à 30 hr[-1].

**Claims**

1.  Process for the purification of 1,1,1,2-tetrafluoroethane (R-134a) containing about 500 to 10,000 ppm by weight of 2-chloro-1,1-difluoroethylene (R-1122), characterised by the placing in contact of the said 1,1,1,2-tetrafluoroethane, in liquid phase, with an activated carbon.

2.  Process according to Claim 1, in which the procedure is carried out at a temperature ranging from about -10 to 100°C and a pressure ranging from about 100 to 860 kPa.

3. Process according to Claim 1 or 2, in which, after the said placing in contact, an R-134a containing less than 10 ppm by weight of 2-chloro-1,1-difluoroethylene is recovered.

4. Process according to one of Claims 1 to 3, in which the activated carbon is used in the form of a fixed bed of particles and the space velocity per hour of the said 1,1,1,2-tetrafluoroethane is about 1 to 30 $h^{-1}$.

**Patentansprüche**

1. Verfahren zur Reinigung von 1,1,1,2-Tetrafluorethan (R-134a), das ungefähr 500 bis 10.000 ppm von 2-Chlor-1,1-difluorethylen (R-1122), bezogen auf das Gewicht, enthält, dadurch gekennzeichnet, daß dieses in flüssiger Phase mit Aktivkohle in Berührung gebracht wird.

2. Verfahren nach Anspruch 1, bei dem man bei einer Temperatur, die sich zwischen -10 und 100 °C und einem Druck, der sich zwischen 100 und 860 kPa bewegt, arbeitet.

3. Verfahren nach Anspruch 1 oder 2, bei dem nach in Berührung bringen ein R-134a gewonnen wird, das weniger als 10 ppm von 2-Chlor-1,1-difluorethylen, bezogen auf das Gewicht, enthält.

4. Verfahren nach einem der Ansprüche 1 bis 3, bei dem die Aktivkohle in Form eines Festbettes der Körner eingesetzt wird, und die Raumgeschwindigkeit des 1,1,1,2-Tetrafluorethans ungefähr 1 bis 30 $h^{-1}$ beträgt.